# EUROPEAN PATENT SPECIFICATION

(11) **EP 4 381 102 B1**
(45) Date of publication and mention of the grant of the patent: **01.07.2026**
(21) Application number: 22758533.8
(22) Date of filing: 02.08.2022
(51) Int. Cl.: C12Q 1/6883

(54) **INFORMATIVE BIOMARKERS OF PORTAL HYPERTENSION**
INFORMATIVE BIOMARKER FÜR PORTALE HYPERTONIE
BIOMARQUEURS INFORMATIFS DE L'HYPERTENSION PORTALE

(30) Priority: 03.08.2021 EP 21382731
(43) Date of publication of application: 12.06.2024
(73) Proprietor: Fundació de Recerca Clínic Barcelona-Institut d'Investigacions Biomèdiques August Pi I Sunyer, 08036 Barcelona (ES); Hospital Clínic de Barcelona, 08036 Barcelona (ES); Consorcio Centro de Investigación Biomédica en Red, 28029 Madrid (ES)
(72) Inventor: GRACIA SANCHO, Jordi, 08036 Barcelona (ES); ORTEGA RIBERA, Martí, 08036 Barcelona (ES); GIBERT RAMOS, Albert, 08036 Barcelona (ES); GARCIA PAGÁN, Joan Carles, 08036 Barcelona (ES); MAGAZ MARTÍNEZ, Marta, 08036 Barcelona (ES)
(74) Representative: ZBM Patents - Zea, Barlocci & Markvardsen
(86) International application number: PCT/EP2022/071655
(87) International publication number: WO 2023/012146

(56) References cited:
- WANG: "Increased expression of urotensin II and GPR14 in patients with cirrhosis and portal hypertension", INTERNATIONAL JOURNAL OF MOLECULAR MEDICINE, vol. 25, no. 6, 21 April 2010 (2010-04-21), GR, XP055874081, ISSN: 1107-3756, DOI: 10.3892/ijmm_00000413
- CHEN JIEGEN ET AL: "Hepatic lipocalin 2 promotes liver fibrosis and portal hypertension", vol. 10, no. 1, 23 September 2020 (2020-09-23), XP055874089, Retrieved from the Internet <URL:https://www.nature.com/articles/s41598-020-72172-7> DOI: 10.1038/s41598-020-72172-7
- KOTANI KOHEI ET AL: "Comprehensive Screening of Gene Function and Networks by DNA Microarray Analysis in Japanese Patients with Idiopathic Portal Hypertension", vol. 2015, 1 January 2015 (2015-01-01), GB, pages 1 - 10, XP055874933, ISSN: 0962-9351, Retrieved from the Internet <URL:https://downloads.hindawi.com/journals/mi/2015/349215.pdf> DOI: 10.1155/2015/349215
- OHTA MASAYUKI ET AL: "Tumor necrosis factor alpha regulates nitric oxide synthase expression in portal hypertensive gastric mucosa of rats", HEPATOLOGY, vol. 27, no. 4, 1 April 1998 (1998-04-01), US, pages 906 - 913, XP055874906, ISSN: 0270-9139, DOI: 10.1002/hep.510270403
- YU SHANSHAN ET AL: "APTR is a prognostic marker in cirrhotic patients with portal hypertension during TIPS procedure", GENE, ELSEVIER AMSTERDAM, NL, vol. 645, 21 December 2017 (2017-12-21), pages 30 - 33, XP085334445, ISSN: 0378-1119, DOI: 10.1016/J.GENE.2017.12.040
- MARGARET ANNE CRAIG ET AL: "Dysregulation of cadherins in the intercalated disc of the spontaneously hypertensive stroke-prone rat", JOURNAL OF MOLECULAR AND CELLULAR CARDIOLOGY, vol. 48, no. 6, 1 June 2010 (2010-06-01), pages 1121 - 1128, XP055081569, ISSN: 0022-2828, DOI: 10.1016/j.yjmcc.2010.01.017
- PALLANTE PIERLORENZO ET AL: "CBX7 Modulates the Expression of Genes Critical for Cancer Progression", PLOS ONE, vol. 9, no. 5, 1 May 2014 (2014-05-01), pages e98295, XP055979881, DOI: 10.1371/journal.pone.0098295

## Description

The present application claims the benefit of European Patent Application EP21382731.4 filed on August 3, 2021.

### Technical Field

The present invention is related to the field of diagnosis. In particular, the present disclosure provides non-invasive methods for the diagnosis of portal hypertension, based on measuring the amount of circulating CBX7-regulating proteins, such as E-cadherin (ECAD) and/or SPINK1 in a bodily fluid sample.

### Background Art

Advanced chronic liver disease (aCLD) is nowadays the 11th most common cause of death globally, with approximately 1.16 million deaths per year. Alcohol abuse, chronic viral hepatitis B or C infection and metabolic-associated fatty liver disease are amongst the most frequent causes of this condition.

Portal hypertension (PH) is one of the main drivers of aCLD-related clinical complications including bleeding from gastro-esophageal varices, ascites, hepatorenal syndrome or hepatic encephalopathy among others, and predisposes the cirrhotic patient to acute-on-chronic liver failure.

PH can be defined as an abnormally increase portal venous pressure - blood pressure in the portal vein and its branches, that drain from most of the intestine to the liver. Portal hypertension is defined as a hepatic venous pressure gradient equal or greater than 5 mmHg.

PH is usually studied as a consequence of the complex cirrhosis syndrome, being accompanied by other confounder factors such as increased stiffness, altered shear stress or immune infiltrate.

Currently, hepatic venous pressure gradient (HVPG) is the most reliable method for assessing PH and allows stratification of patients with normal pressure (NP, HVPG ≤ 5mmHg), PH (HVPG > 5mmHg) and clinically significant PH (CSPH, HVPG ≥10mmHg). In patients with more than 10 mmHg and especially above 12mmHg, clinical complications appear, being at higher risk of hepatic decompensation and death. However, HVPG measurement is an invasive procedure with some limitations including availability and affordability in all centres, requirement of high-technical skills, patient discomfort and improbable but existing risk of severe complications such as bleeding. Recently, transient elastography has been in the spotlight as an alternative technique for assessing PH and fibrosis. Still, inter-professional interpretation of the data, lobule bias and high costs for primary attention centres represent the main hindrances in the appropriate diagnostic and monitoring of PH.

Liu et al, INTERNATIONAL JOURNAL OF MOLECULAR MEDICINE, vol. 25, no. 6, 21 April 2010, relates to increased expression of urotensin II and GPR14 in patients with cirrhosis and portal hypertension.

Therefore, there is still the need of ubiquitous and reliable non-invasive tests for identifying and precisely stratifying patients suffering from PH.

### Summary of Invention

The present invention is defined by the appended claims.

The present inventors have found that pathological hydrodynamic pressure induces liver sinusoidal endothelial cells (LSEC) disfunction. In particular, they have found that chromobox 7 (CBX7), mainly expressed in LSECs, was a pressure-related factor.

As it is shown below, the inventors have surprisingly found that CBX7 downregulation correlates with PH. Tables 3 and 4 below provides the CBX7 liver expression in the discovery and validation cohorts: these data show that CBX7 exhibits an excellent diagnostic ability, being above 0.965 with a sensitivity of 100% and a specificity of at least 75% and 90%, respectively.

The inventors further found that CBX7-regulated circulating proteins such as E-cadherin (ECAD) and serine peptidase inhibitor, Kazal type I (SPINK1), were differentially expressed in plasma samples from healthy subjects and subjects suffering PH (above 5 mmHg). Table 5 shows that both markers are upregulated when the subject suffers from PH.

Not only that, but also, the present inventors have found that these CBX7-regulated circulating proteins are also differentially expressed in CSPH (See Table 6 below). Altogether, both ECAD and SPINK1, are non-invasive markers which provides robust and precisely identification of a patient suffering from PH.

Thus, in a first aspect the present invention provides a method as defined in appended claim 1, of diagnosis portal hypertension (PH) in a subject, the method comprising the steps of: (a) measuring the amount of the gene expression product of the marker ECAD or of the markers E-cadherin (ECAD) and SPINK1 in an isolated fluid sample from the subject; (b) comparing the amount with a reference value; and (c) diagnosing the portal hypertension when the measured amount is higher than the reference value.

As stated above, HVPG is the standard technique for assessing PH but, despite being the most trustable procedure, it has some limitations such as universal coverage, need for skilful professionals, patient discomfort and risk of complications.

The present invention means a great advance in the diagnosis and appropriate monitoring of the disease: two markers, which can be easily quantified in a fluid sample, provide a robust information to classify a subject as suffering or not PH.

Not only that, but also, the present inventors have also found that these markers provide information of the severity of the disease: the highest the amount of the marker is, the highest is the pressure within the portal vein, which will indicate that the subject is in advance phase of the PV, suffering a clinically significant PH.

In view of the above, the ECAD and SPINK1, not only provide a non-invasive method for identifying subjects suffering PH (when they are overexpressed), but also they are so "sensitive" to the pressure within the portal vein, that can precisely inform of the real situation of subject, depending on the amount measured. Therefore, these markers provide robust diagnostic and monitoring information to the physician: the highest amount, the highest the pressure within the portosinusoidal system.

Thus, in a further aspect the present invention provides a method of prognosis PH, the method comprising measuring the amount of the gene expression product of the marker ECAD or of the markers ECAD and SPINK1 in an isolated fluid sample from the subject, at least at two different temporal points; wherein if there is an increase in the amount of expression product, this indicates that there is a bad prognosis, particularly this is indicative that the subject is at risk of progressing to severe portal hypertension or decompensation.

In a further aspect the present invention further provides a method for stratifying a subject as normal portal pressure (NP), as suffering from non-clinically significant PH (NCSPH) or as suffering clinically significant PH (CSPH), the method comprising the steps of:
(i) measuring the amount of gene expression product of the marker ECAD or of the markers selected from ECAD and SPINK1 in an isolated fluid test sample of the subject;
(ii) obtaining reference control values V1 and V2, wherein:
   V1 is calculated measuring the amount of the marker(s) in a group of samples from subjects with a portal pressure equal or lower than 5 mmHg; and
   V2 is calculated measuring the amount of the marker(s) in a group of samples from subjects with a portal pressure equal or lower than 10 mmHg, and higher than 5 mmHg;
(iii) comparing the measured amount of step (i) with the reference control values V1 and V2, wherein:
   if the measured value of (i) is equal or below V1, the subject is identified as "normal portal pressure";
   if the measured value of (i) is between V1 and V2, the subject is identified as suffering NCSPH; and
   if the measured value of (i) is equal or above V2, then the subject is identified as suffering CSPH.

The plasmatic factors ECAD and SPINK1 allow the correct detection and classification of patients with NCSPH or CSPH in a routine blood test. This novel analytical approach could be easily implemented into decision making for patients with chronic liver disease (CLD), for instance, who may avoid unnecessary HVPG measurement if the plasmatic values for the ECAD alone or in combination with SPINK1 is above the proposed cut-off values. But also, provides therapeutic advantages, in the managing of the most appropriate therapeutic approach.

Therefore, in a further aspect, the present invention provides a method of deciding or recommending whether to initiate a medical regimen of a subject suspicious of suffering PH (either as "NCSPH" or "CSPH"), which method comprises the steps of:
Step a:
   a.1) diagnosing the subject as suffering; or, alternatively,
   a.2) prognosing the subject; or, alternatively,
   a.3) stratifying the subject as NCSPH or CSPH,
   following any of the above provided methods of the invention, and
Step b: deciding or recommending to initiate the medical regimen if the subject is diagnosed as suffering PH or stratified as NCSPH or CSPH.

The medical regimen in case of portal hypertension involves lactulose, enemas, and use of antibiotics such as rifaximin, neomycin, vancomycin, and the quinolones. This regimen also considers in those more severe stages surgery interventions based, for instance, in portosystemic shunts, and fluoroscopic image of transjugular intrahepatic portosystemic shunt (TIPS). Diagnosis of patients with NCSPH, which is currently difficult, is highly relevant since these patients may be at the onset or initial stages of the disease. The only specific treatment prescribed to these individuals, besides removal of the etiological factor and lifestyle recommendations, is (non-selective) beta-blockers. In the case of patients with CSPH, (non-selective) beta-blockers and/or statins is currently the recommended therapy to ameliorate portal hypertension. In patients with clinical complications such as ascites, peritonitis and hepatic encephalopathy additional treatments may be required (diuretics, antibiotics or dialysis).

In a further aspect, the present invention provides a method for determining the efficacy of a medical regimen in a patient already diagnosed of PH (either as "NCSPH" or "CSPH"), the method comprising the steps of:
(a) measuring the amount of the gene expression product of the marker ECAD or the markers ECAD and SPINK1 in an isolated fluid sample from the patient prior to the administration of the medical regimen;
(b) measuring the amount of the gene expression product of the one or both markers in an isolated fluid sample from the patient once started the administration of the medical regimen; and
(c) comparing the levels measured in steps (a) and (b), in such a way that if the amount measured in step (b) is lower than the amount measured in step (a), it is indicative that the medical regimen is effective in the treatment of the condition.

In a further aspect, the present invention provides the use of ECAD alone or in combination with SPINK1 in the diagnosis and prognosis of PH; in the stratification of a subject as normal portal pressure, as suffering from non-clinically significant PH (NCSPH) or as suffering clinically significant PH (CSPH); in a method of deciding or recommending whether to initiate a medical regimen of a subject suspicious of suffering PH; or for determining the efficacy of a medical regimen in a patient already diagnosed of PH.

In a further aspect, the present invention provides the use of means for performing any of the methods provided by the present invention.

Present disclosure further relates to a method of diagnosing, prognosing or stratifying a subject suffering from PH, the method comprising measuring the amount of CBX7 in a test sample of the subject. Said method is not part of the claimed invention.

Present disclosure further relates to the use of CBX7 for the diagnosis, prognosis or stratification of a subject suffering from PH. Said use is not part of the claimed invention.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The present disclosure provides non-invasive methods for the diagnosis, monitoring and stratification of subjects suffering from PH, based on determining the amount of ECAD alone or in combination with SPINK1 in an isolated bodily fluid sample of the subject.

In the context of the present invention, "portal hypertension" (PH) is understood as a blood pressure in the portal vein and its branches, that drain from most of the intestine to the liver, equal or higher than 5 mmHg. PH can be measured using any commercial device. An illustrative example is the balloon-tipped catheter used in the Examples provided below, but the skilled person, using their general knowledge can use other alternative devices with the same end. The election of one or other device does not significantly affect to the pressure measure.

In the context of the present invention, "normal portal pressure" (NP) subject, means that the portal vein pressure is below 5 mmHg; "non-clinically significant PH" (NCSPH), means that the portal vein pressure is equal or higher than 5 but lower than 10 mmHg; and "clinically significant PH" (CSPH) means that the portal vein pressure is equal or above 10 mmHg.

E-cadherin (ECAD) is a cell adhesion molecule expressed by different cell types that can be found in the plasma as soluble e-cadherin, resulting from the proteolytic cleavage of the cell surface ECAD and indicating several processes including a disruption in cell-cell interaction and increases in cell migration and proliferation. SPINK1 has been reported to increase in the serum of different cancers and to be linked to the progression of HBV-related diseases.

The term "diagnosis" is known to the person skilled in the art. As used herein "diagnosis" is understood as becoming aware of a particular medical condition, syndrome, complication; the determination of the nature of the disease or condition; or the distinguishing of one disease or condition from another. It refers both to the process of attempting to determine or identify the possible disease or condition, and to the opinion reached by this process. A diagnosis, in the sense of diagnostic procedure, can be regarded as an attempt at classification of an individual's condition into separate and distinct categories that allow medical decisions about treatment and prognosis to be made. Subsequently, a diagnostic opinion is often described in terms of a disease or other condition. However, a diagnosis can take many forms. It might be a matter of detecting the presence and naming the disease, lesion, dysfunction or disability. It might be an exercise to attribute a category for management or for prognosis. It may indicate either degree of abnormality on a continuum or kind of abnormality in a classification.

"Prognosis" as used herein refers to the prediction of the probable progression and outcome of a disease.

The term "amount of gene expression product" as used herein have the same meaning as, and can be interchanged by, "level of gene expression product". As used herein, "gene expression product" or "expression gene product" or "expression product" refers to the mRNA (transcript) generated when a gene is transcribed, or the protein generated when a gene is transcribed and translated.

In the present invention, the term "reference value" referred in the methods of the invention has to be understood as a predefined value of a given molecular marker, which is derived from the levels of said molecular marker in a sample or group of samples. The samples are taken from a subject or group of subjects wherein the presence, absence, stage, or course of the disease has been properly performed previously. This value is used as a threshold to discriminate subjects wherein the condition to be analysed is present from those wherein such condition is absent, to determine the stage of the disease, or the risk of developing or of being suffering from a thrombosis condition, among others. This reference control level is also useful for determining whether the subject has to initiate a medical regimen.

Methods for obtaining the reference value from the group of subjects selected are well-known in the state of the art (Burtis C. A. et al., 2008, Chapter 14, section "Statistical Treatment of Reference Values"). In a particular case "reference value" is a cut-off value defined by means of a conventional ROC analysis. As the skilled person will appreciate, optimal cut-off value will be defined according to the particular applications of the diagnostic or prognostic method: purpose, target population for the diagnosis or prognosis, balance between specificity and sensibility, etc.

The subject or subjects from whom the "reference value" is derived may include subject/s wherein the condition is absent, subject/s wherein the condition is present, or both. The skilled person in the art, making use of the general knowledge, is able to choose the subject or group of subjects more adequate for obtaining the reference control level for each of the methods of the present invention. In an embodiment of the present invention, optionally in combination with any of the embodiments provided above or below, the reference value referred in the diagnostic method is taken from a group of subjects which do not suffered PH. The main criterium to consider the subject as not suffering PH is that the HVPG value is below 5 mmHg, when measured using any of the devices already available in the state of the art.

In the present invention, the term "HVPG" refers to the hepatic venous pressure gradient, and it is determined as the difference between the Wedged hepatic venous pressure in the main right or middel hepatic vein (WHVP) and the free hepatic venous pressure (FHPV). The venous pressure can be measured using any of the devices already available in the market, following manufacturer's instructions. For example, as shown below, the pressure can be measured using a 7F balloon-tipped catheter (such as "Fogarty" Edwards Lifesciences LLC, CA). All measurements can be taken by triplicate and averaged to obtain the baseline HVPG. Real-time measurements on hepatic venous pressure values were obtained in each case in a multichannel recorder (GE Healthcare, Milwaukee, WI).

In an alternative embodiment of the present invention, optionally in combination with any of the embodiments provided above or below, the reference value of the prognosis method corresponds to the measure of the marker(s) in a previous moment of time (in terms of days, weeks or months).

When combining ECAD and SPINK1, the better predictive power was obtained. ECAD + SPINK1 were able to discriminate patients with NP or PH with a sensitivity of 93.1% and specificity of 83.3%. But also, was able to discriminate patients CSPH vs NCSPH with a 91.4% sensitivity and 63.6% specificity.

Therefore, in one embodiment of the methods of the present invention, optionally in combination with any of the embodiments provided above or below, the amount of both, the ECAD and SPINK1 markers, is measured.

In another embodiment of the methods of the present invention, optionally in combination with any of the embodiments provided above or below, the fluid test sample is selected from the group consisting of whole blood, plasma, serum, urine and saliva. Particularly, the fluid sample is plasma, blood or serum.

In another embodiment of the methods of the present invention, optionally in combination with any of the embodiments provided above or below, the sample is isolated from a subject already diagnosed of suffering a liver disease, such as chronic liver disease, Budd-Chiari syndrome or portal vein thrombosis. The chronic liver disease can be in an earlier or advance stage. In one embodiment the chronic liver disease is chronic cirrhosis due to any aetiology including alcoholic abuse, viral hepatitis or metabolic liver disease. Additionally, combining the expression of these CBX7-related targets with other scores or liver-derived parameters (i.e. platelet count, AST or bilirubin) will increase the prognostic value of the herein presented data.

Thus, in another embodiment of the methods of the present disclosure, optionally in combination with any of the embodiments provided above or below, the methods further comprise one or more of the following steps:
i) measuring the level of one or more of the following markers: cholesterol; inflammatory biomarkers such as IL-Iβ, IL-IRa, Fas-R, VCAM1 , TNF-β, HSP-70, IL-18, TLR9, lymphotoxin-β, glutamine, glutamine synthase, HSP-27, HSP-60, HSP-110, grpl70, hyaluronan, homeocysteine, or angiotensin- II; and /or
ii) further correlation with demographic and clinical laboratory parameters selected from the group consisting of age, model for end-stage liver diseases (MELD), Child-Pugh Score (CPS), platelets, alanine aminotransferase (ALT), aspartate aminotransferase (AST), platelet count, prothrombin time (PT/INR), liver stiffness, and at-risk alcohol use for indicating EGD to the patient or not indicating EGD for the patient.

Various mathematical approaches for correlating biomarker panels based on several markers and parameters, are well-known in the art. And mainly rely on statistical analysis known as multivariate classification or supervised learning. Exemplary methods, such as a Pearson's correlation coefficient, multiple linear regression analysis, threshold-based methods, logistic regression analysis, tree-based methods, and Support Vector Machine (SVM), can be applied to correlate biomarker panels with a clinical diagnosis, prognosis, stratification or indication for further treatment. Generalized additive models also allow one to combine data with patient clinical information to predict an outcome. Furthermore, several other methods, e.g., a Bayesian network on gene expression microarray data, perform well in proteomics based biomarker detection. Additional well-known data preprocessing and data normalization steps can be implemented. Additional computational methods and randomization techniques, such as a permutation test, cross-validation and bootstrapping, can help to evaluate and validate the performance and correlation. A more detailed discussion of methods for defining, classifying and performance validation of a biomarker panel are described in Robin *et al.,* 2009.

In another embodiment of the methods of the invention, optionally in combination with any of the embodiments provided above or below, the level of expression of the gene is determined by measuring or determining the amount of corresponding mRNA or protein (e.g., full-length protein product or a proteolytic fragment thereof), depending on the detection technique to be used.

In another embodiment of the methods provided by the present invention, the level of the protein markers or fragments thereof is determined by a quantitative test selected from the group consisting of an immunological test, bioluminescence, fluorescence, chemiluminescence, electrochemistry and mass spectrometry.

In one embodiment of the invention, the level of expression is determined by immunochemistry.

The term "immunochemistry" as used herein refers to a variety of techniques for detecting antigens (usually proteins and peptides, and in the present case any of the proteins listed above alone or in combination) in a sample by exploiting the principle of antibodies binding specifically to said antigens. Visualising an antibody-antigen interaction can be accomplished in a number of ways. In the most common instance, an antibody is conjugated to an enzyme, such as peroxidase, that can catalyse a colour-producing reaction. Alternatively, the antibody can also be tagged to a fluorophore, such as fluorescein or rhodamine. The immunochemistry technique can be direct or indirect. The direct method is a one-step staining method and involves a labelled antibody (e.g. FITC-conjugated antiserum) reacting directly with the antigen. While this technique utilizes only one antibody and therefore is simple and rapid, the sensitivity is lower due to little signal amplification, such as with indirect methods, and is less commonly used than indirect methods. The indirect method involves an unlabelled primary antibody (first layer) that binds to the target antigen in the sample and a labelled secondary antibody (second layer) that reacts with the primary antibody. This method is more sensitive than direct detection strategies because of signal amplification due to the binding of several secondary antibodies to each primary antibody if the secondary antibody is conjugated to the fluorescent or enzyme reporter.

Further amplification can be achieved if the secondary antibody is conjugated to several biotin molecules, which can recruit complexes of avidin-, streptavidin or Neutravidin-enzyme. The indirect method, aside from its greater sensitivity, also has the advantage that only a relatively small number of standard conjugated (labelled) secondary antibodies needs to be generated. With the direct method, it would be necessary to label each primary antibody for every antigen of interest. It must be borne in mind that immunochemistry techniques can also be used to detect certain nucleic acid sequences if a tagged nucleic acid probe (designed to specifically bind to a certain target nucleic acid sequence) can later on be detected with a labelled antibody. Thus, the detection of the protein could be performed by using a tagged nucleic acid designed to bind a specific sequence of the target protein RNA, and then detecting said tagged nucleic acid with a labelled antibody which selectively binds to the tag.

Immunoassay procedures suitable include enzyme-linked immunosorbent assays (ELISA), enzyme immunodot assay, agglutination assay, antibody-antigen-antibody sandwich assay, antigen-antibody-antigen sandwich assay, immunocromatography, or other immunoassay formats well-known to the ordinarily skilled artisan.

In one embodiment, in combination with any of the embodiments provided above or below, the level of expression of protein(s) is determined by an immunoassay.

In another embodiment, in combination with any of the embodiments provided above or below, the level of expression of protein is determined by ELISA.

All the above embodiments, related to the methods of the invention, are also embodiments of the uses provided by the present invention, too.

In one aspect, the invention provides the use of means for performing any of the methods of the invention. In one embodiment, the means are antibodies or fragments thereof, which can form part of a kit. The kit may additionally comprise means (additives, solvents) to visualize the antibody-protein interactions.

The term "antibody or a fragment thereof able to bind to the target protein(s)" is to be understood as any immunoglobulin or fragment thereof able to selectively bind the target protein. It includes monoclonal and polyclonal antibodies. The term "fragment thereof encompasses any part of an antibody having the size and conformation suitable to bind an epitope of the target protein. Suitable fragments include F(ab), F(ab') and Fv. An "epitope" is the part of the antigen being recognized by the immune system (B-cells, T-cells or antibodies).

The antibodies used for specific detection can be polyclonal or monoclonal. There are well known means in the state of the art for preparing and characterizing antibodies. Methods for generating polyclonal antibodies are well known in the prior art. Briefly, one prepares polyclonal antibodies by immunizing an animal with the protein; then, serum from the immunized animal is collected and the antibodies isolated. A wide range of animal species can be used for the production of the antiserum. Typically, the animal used for production of antisera can be a rabbit, mouse, rat, hamster, guinea pig or goat.

Moreover, monoclonal antibodies (MAbs) can be prepared using well-known techniques. Typically, the procedure involves immunizing a suitable animal with the protein associated with the disease. The immunizing composition can be administered in an amount effective to stimulate antibody producing cells. Methods for preparing monoclonal antibodies are initiated generally following the same lines as the polyclonal antibody preparation. The immunogen is injected into animals as antigen. The antigen may be mixed with adjuvants such as complete or incomplete Freund's adjuvant. At intervals of two weeks, approximately, the immunization is repeated with the same antigen.

The *in vitro* methods and uses of the invention provide an information in terms of diagnosis, prognosis, medical regimen and stratification. In one embodiment, the methods further comprise the steps of (1) collecting the information about the diagnostic, prognostic, medical regimen and/or stratification, and (2) saving the information in a data carrier.

In the sense of the invention a "data carrier" is to be understood as any means that contain meaningful information data for the diagnosis or prognosis of endometrial carcinoma, such as paper. The carrier may also be any entity or device capable of carrying the prognosis data. For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means. When the prognosis data are embodied in a signal that may be conveyed directly by a cable or other device or means, the carrier may be constituted by such cable or other device or means. Other carriers relate to USB devices and computer archives. Examples of suitable data carrier are paper, CDs, USB, computer archives in PCs, or sound registration with the same information.

In a further aspect, not part of the claimed invention, the present disclosure relates to a treatment method comprising (a) measuring the amount of the gene expression product of one or both of the markers E-cadherin (ECAD) and SPINK1 in an isolated fluid sample from the subject; to identify the patient as having portal hypertension (PH), and (b) administering a treatment to the patient having PH, wherein the treatment is selected from beta-blockers, lactulose, enemas, diuretics , antibiotics (such as rifaximin, neomycin, vancomycin, and the quinolones), surgery, statins, dialysis, and combinations thereof.
not part of the claimed invention, the present disclosure relates to a treatment method comprising (a) measuring the amount of the gene expression product of one or both of the markers E-cadherin (ECAD) and SPINK1 in an isolated fluid sample from the subject; to identify the patient as having non-clinically significant PH (NCSPH), and (b) administering a treatment to the patient having NCSPH, wherein the treatment is beta-blockers.
not part of the claimed invention, the present disclosure relates to a treatment method comprising (a) measuring the amount of the gene expression product of one or both of the markers E-cadherin (ECAD) and SPINK1 in an isolated fluid sample from the subject; to identify the patient as having clinically significant PH (CSPH), and (b) administering a treatment to the patient having CSPH, wherein the treatment is selected from statins, beta-blockers, surgery, and combinations thereof.

In a further aspect, the disclosure provides a method of detecting a gene expression product of one or both of the markers E-cadherin (ECAD) and SPINK1 in an isolated fluid sample from the subject, said method comprising (a) obtaining a plasma sample from a human patient; and (b) detecting whether a gene expression product of one or both of the markers ECAD and SPINK1 is present in the plasma sample by contacting the plasma sample with an anti-ECAD and/or an anti-SPINK1 antibody and detecting binding between the gene expression product of one or both of the markers ECAD and/or SPINK1 and the antibody.

Throughout the description and claims the word "comprise" and variations of the word are not intended to exclude other technical features, additives, components, or steps. Furthermore, the word "comprise" and its variations encompasses the term "consisting of. Additional objects, advantages and features of the invention will become apparent to those skilled in the art upon examination of the description or may be learned by practice of the invention. The following examples are provided by way of illustration, and they are not intended to be limiting of the present invention. Furthermore, the present disclosure covers all possible combinations of particular and preferred embodiments described herein.

### Examples

### Materials and Methods

### Animals

Male Wistar rats were kept at the University of Barcelona Faculty of Medicine facilities, housed three per cage, under controlled environmental conditions (19.7 ± 2°C, 52 ± 5% humidity, 12 hours light/dark cycle) and free access to standard rodent food pellets and water. All the experimental procedures were approved by the Laboratory Animal Care and Use Committee of the University of Barcelona and performed in accordance with the European Community guidelines for the protection of animals used for experimental and other scientific purposes (EEC Directive 86/609).

### Isolation of hepatic cells

Primary hepatocytes and non-parenchymal cells from healthy rats weighing 300-350 g were isolated using the 4 in 1 protocol as previously reported (Fernández-Iglesias, A. *et al*., 2019). Briefly, liver was perfused, digested with 0.015% collagenase A (103586, Roche, Sant Cugat del Vallès, Spain) and mechanically disaggregated obtaining a multicellular suspension. Hepatocytes were purified by low-speed centrifugation (5 min at 50g) and non-parenchymal cells were separated using a three-phase iodixanol (Optiprep^{™}, Sigma-Aldrich, Saint Louis, MO, USA) density gradient centrifugation. Afterwards, the upper interphase, that contained the hepatic stem cells (HSCs), was directly seeded in Iscove Modified Dulbecco Media (IMDM) culture media at 37°C 5% CO₂ in the incubator. The lower interphase, enriched in liver sinusoidal endothelial cells (LSECs) and Kupffer *cells* (KCs), was further purified by differential adherence time to non-coated substrates. After 30 min of culture of both cell types, KCs were attached to the culture plate while LSECs were found in the supernatant. Highly pure and viable LSECs cells (as described in Fernández-Iglesias A et al., 2019) were seeded on conventional culture plates or on liver-on-a-chip devices as described below

### Hydrodynamic pressure on a liver-on-a-chip

The study of hydrodynamic pressure *in vitro* was performed using an advanced sinusoid-mimicking microfluidic device named as Exoliver. The details of its fabrication, features and suitability for sinusoidal studies have been previously reported by Ortega-Ribera, M. *et al.* 2018.

Primary cells isolated from healthy rats were exposed to physiological (1 mmHg) or pathological (12 mmHg) pressures within the liver-on-chip device for 48h. Laminar shear stress stimulus on LSECs cultures was maintained at 1.15 dyn/cm² (Ortega-Ribera, M. *et al*., 2019).

Desired pressure within the device was ensured by modulating outflow height relative to LSECs culture (Bernouilli's equation) and according to 1 mmHg = 1.36 cm H₂O. Real-time assessment of pressure within the bioreactor was routinely performed in independent experimental settings using a pressure probe both at the inflow and outflow of the device connected to a Powerlab (4SP). Data was displayed into a LabChart v5.5.6 software file.

Device configuration and culture was performed as previously described by Ortega-Ribera and colleagues (Ortega-Ribera, M. *et al.* 2019), with minor modifications. Briefly, LSECs were cultured on a hydrophilic biocompatible polytetrafluoroethylene microporous membrane (Omnipore, Millipore, USA), and hepatocytes, KCs and HSCs at a lower layer, immediately below and in contact with the endothelial fraction of the culture. Microfluidic cultures were maintained at 37°C and 5% CO₂, with 43 ml of a recirculating media comprising ²⁰; DMEM/F12 was supplemented with 2.97% dextran (31392; Sigma-Aldrich, Darmstadt, Germany), 2% fetal bovine serum (04-001-1A; Biological Industries, Kibbutz Beit-Haemek, Israel), 1% penicillin-streptomycin (10378-016, Biological Industries), 1% endothelial cell growth supplement (BT-203; Biomedical Technologies, Kandel, Germany), 1% heparin (H3393; Sigma-Aldrich), 1% L-glutamine (25030-024; Gibco, Dublin, Ireland), 1% amphotericin B (03-029-1C; Biological Industries), 1 nM dexamethasone (D4902; Sigma-Aldrich), 10 ng/ml Epidermal Growth Factor (E4127; Sigma-Aldrich), 1.5 nM glucagon (16941-32-4, Novo Nordisk, Plainsboro, NJ, USA), 15 nM hydrocortisone (H0888, Sigma-Aldrich), and 1 µM insulin (Humulin S, Lilly S.A.).

### mRNA sequencing

Primary LSECs transcriptome profile under physiological or pathological hydrodynamic pressures was examined by mRNA sequencing. Briefly, mRNA was isolated using the RNeasy^{®} Micro Kit (Qiagen, Hilden, Germany) following manufacturer's instructions. The sequencing library was prepared using 25 ng of total RNA by a Universal Plus mRNA-Seq NuGEN (0508, 9133, 9134, Tekan, Leek, The Netherlands). Single-end mRNA-seq was performed in the Illumina platform HiSeq2500. The dataset is available at www.shiny.lvbrg.barcelona/lsec_PH. Venn diagrams were performed using the Venny2.1 free online software (by Juan Carlos Oliveros, BioinfoGP, CNB-CSIC). Canonical pathways analysis was performed using the Ingenuity Pathways Analysis software from Qiagen (content version: 49932394). Transcriptomics data from LSECs isolated from preclinical models of aCLD (CCl₄; thioacetamide, TAA; and common bile duct ligation, cBDL) and alcohol associated cirrhotic patients were obtained from Manicardi, N. and colleagues (Manicardi, N. *et al.,* 2021).

### Gene Expression Analysis

Total RNA was extracted and purified with TRItidy G^{™} (A4051,0200, Panreac, Castellar del Vallès, Spain) and TRIzol:chloroform for liver tissues or using Qiazol lysis reagent and RNeasy^{®} Micro Kit (Qiagen) for primary cells according to the manufacturer instructions. RNA yield was quantified by Nanodrop ND-1000 spectrophotometer (NanoDrop Technologies, Wilmington, DE, USA). A total amount of 0.5 µg (tissue) and 0.15 µg (cells) of total RNA was reverse transcribed using a High-Capacity cDNA Reverse Transcription Kit (Applied Biosystems) in a thermal cycler (Eppendorf AG 22331, Hamburg, Germany), and qPCR was performed in a 7900HT Fast Real-Time PCR System (Thermofisher), using the TaqMan Universal PCR Master Mix (Applied Biosystems).

RNA expression levels were normalized following the 2-ΔΔCt method, with beta actin (Actb) or glyceraldehyde-3-phosphate dehydrogenase (Gapdh) as housekeeping genes. The following commercial Thermofisher TaqMan^{™} gene expression assay (FAM) probes were used: chromobox 7 (*CBX7,* Rn01506264_m1), Fc-gamma receptor IIb (*CD32b,* Rn00598391 m1), stabilin 2 (*Stab2,* Rn01503539_m1), *Gapdh* (Rn01775763_g1), hsa-miR-181a-5p (000480) and U6 snRNA (001973). For human: *CBX7* (Hs00545603_m1) and Actb (Hs99999903_m1).

### Patients

Three different cohorts of patients from the Hospital Clinic of Barcelona (Spain) were used for this study; two including HVPG measurement and liver biopsy collected with an 18-G Tru-cut needle during PP measurement (discovery and validation cohort) and one including HVPG and blood sample collected via systemic venous access just before PP measurement (biomarker cohort).

The discovery cohort (31 subjects) was a retrospective one consisting in 12 healthy volunteers and 19 portal hypertensive cirrhotic patients due to HCV infection with similar ages and with the majority of patients with a Child Pug A grade (n= 16). Among the 19 portal hypertensive patients: 6 patients had a HVPG< 10 mmHg, 5 patients had 10<HVPG<12, and 8 patients had a HPVG> 12 mmHg.

The validation cohort was prospectively collected and comprised 11 subjects without PH (5 healthy volunteers, 5 patients with portal cavernomatosis and 1 patient with unspecific vascular aberrations), 41 portal hypertensive cirrhotic patients of HCV (n= 16) and OH (n= 25) aetiologies, and 14 HCV cirrhotic patients 12 months after receiving antiviral treatment:

**TABLE 1**

| | ***Group*** | ***N*** | ***HVPG (mmHg)*** |
|---|---|---|---|
| *Discovery cohort* | healthy | 12 | 4 ± 0.8 |
| | CLD | 19 | 12 ± 4.5 |
| *Validation cohort* | healthy | 11 | 3 ± 1.3 |
| | CLD | 41 | 15.6 ± 7.6 |
| | HCV + antiviral | 14 | 10.2 ± 3.6 |

The biomarker cohort was a prospective one with 18 normotensive healthy volunteers and 46 patients with CLD-derived portal hypertension.

Patients included in all cohorts underwent HVPG measurement due to clinical recommendation.

The protocol of this study was reviewed and approved by the Ethical and Clinical Investigation Committee of the Hospital Clinic of Barcelona and was in according with the Helsinki Declaration of 1975, as revised in 1983. Written informed consent was obtained from each patient.

### HVPG measurement

HVPG measurement was performed at the Barcelona Hepatic Hemodynamic Unit as described (Bosch, J. *et al.,* 2008). In short, a 7F balloon-tipped catheter ("Fogarty" Edwards Lifesciences LLC, CA) was guided into the main right or middle hepatic vein for measurements of wedged and free hepatic venous pressures. The HVPG resulted from the difference between both measurements.

All measurements were taken by triplicate and averaged to obtain the baseline HVPG. Permanent tracings were obtained in each case in a multichannel recorder (GE Healthcare, Milwaukee, WI).

### Plasma collection

For the biomarker cohort, blood was drawn from the subjects forming each one of the above cohorts, collected in BD Vacutainer^{®} K2 EDTA tubes (KFK286, Becton Dickinson), immediately kept at 4°C, centrifuged at 1300g for 10min at 4°C to obtain the plasma, and further centrifuged at 3000g for 15 min at 4°C to remove contaminating platelets. Plasma was aliquoted and stored in the IDIBAPS Biobank at -80°C following the internal agreement policy until used.

### Enzyme linked immunosorbent assay (ELISA)

Levels of E-cadherin (ECAD) and serine protease inhibitor Kazal-type 1 (SPINK1) in plasma was measured by ELISA following manufacturer's instructions. ECAD (ab233611) kits were purchased from Abcam (Cambridge, United Kingdom) and SPINK1 (DY7496-05) kits were purchased from R&D Systems (Abingdon, United Kingdom).

### Statistical analyses

Data are presented as mean ± standard deviation (SD) based on at least three independent experiments. Statistical analysis was performed using One-Way ANOVA or Student's t-test for parametric variables or Mann-Whitney U test for non-parametric tests. The correlations between *CBX7* expression and HVPG were calculated by Chi-squared test and Spearman's rank correlation. Differences were considered statistically significant for a p-value <0.05. Areas under the receiver operating characteristic (AUROC) curve and cut off values for the best specificity and sensitivity established, positive predictive value (PPV) and negative predictive value (NPV) were calculated. Statistical analyses were performed using GraphPad Prism 8.0.2 (GraphPad Software, Inc; San Diego, CA, USA) or SPSS (IBM, Chicago, IL, USA) software.

### Results

### Pathological hydrodynamic pressure induces LSECs dysfunction

The present inventors performed, a bulk RNA sequencing of LSECs cultured under physiological (1 mmHg) or pathological (12 mmHg) pressure in order to elucidate whether hydrodynamic pressure had a pathological effect on LSECs.

Transcriptomic analysis revealed 206 deregulated genes (197 downregulated and 9 upregulated) with a fold change ≥ 3 and p-value < 0.05 in response to pathological pressure. Interestingly, the inventors confirmed a global detrimental impact in LSEC homeostatic functions in view of the differentially expressed genes (DEG) identified, implied in different molecular processes categories, involved in LSECs metabolism, cell death and survival, proliferation, angiogenesis/remodelling, and inflammation.

This is the first time that it is reported the correlation between hydrodynamic pressure and LSEC dysfunction.

Table 2 shows the top 50 de-regulated genes in response to pathological hydrodynamic pressure.

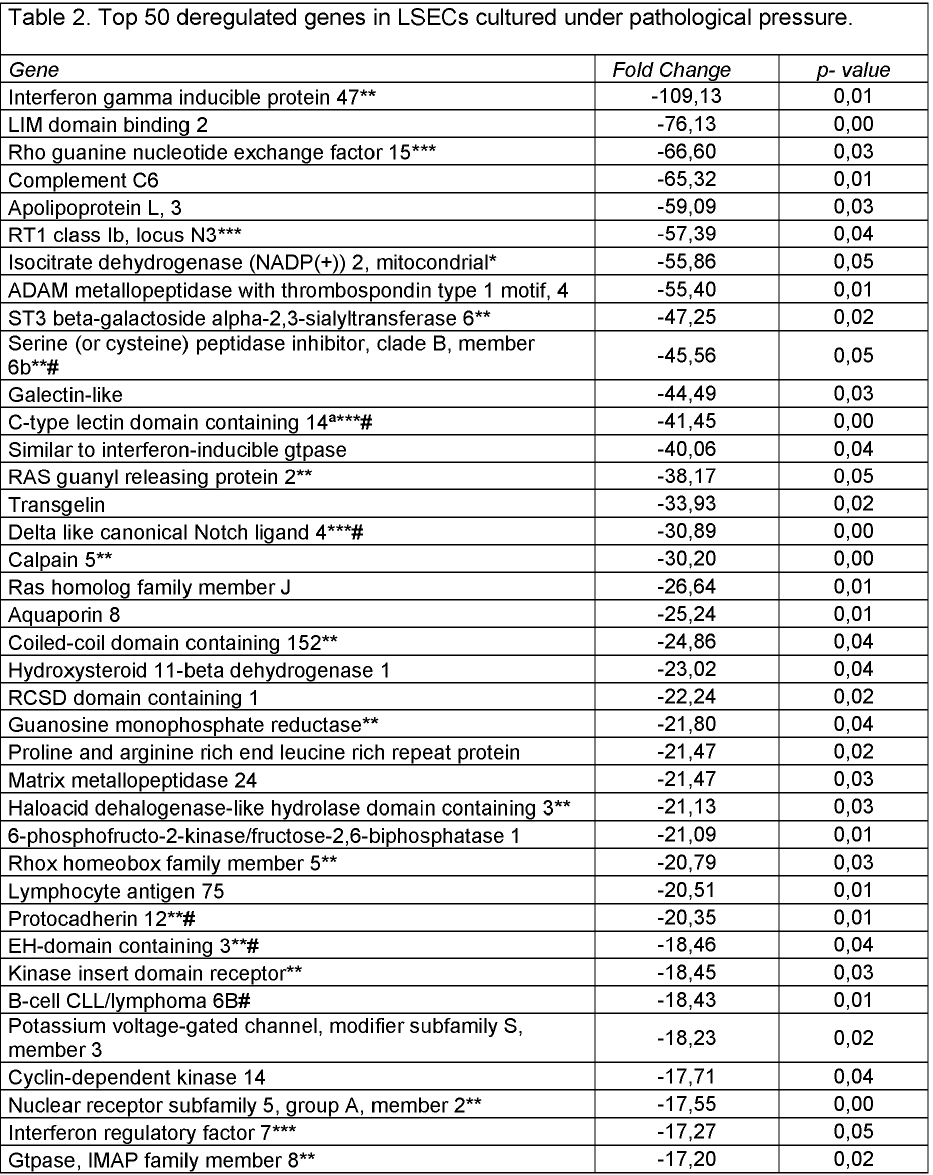

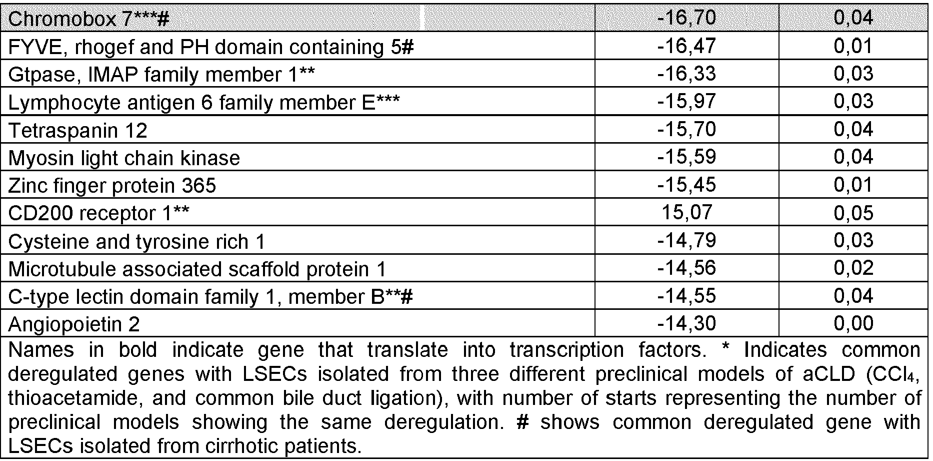

These are pressure-specific DEG which can provide information of LSEC dysfunction due to hydrodynamic pressure.

As increased pressure is part of the complex and multifactorial pathophysiology of aCLD, the inventors then compared the pressure specific DEG identified as explained above, with those reported as differentially expressed in LSECs isolated from 3 different preclinical models of aCLD: chronic CCl₄, chronic TAA and cBDL (Manicardi N. et al, 2021). Additionally, pressure-specific DEG were also compared to those from primary LSECs isolated from cirrhotic patients (hLSEC).

Comparison analysis between these groups showed that Chromobox 7 (*CBX7*) was the only gene with transcription regulation activity that was also downregulated in LSECs isolated from the three preclinical models of aCLD and in LSECs isolated from cirrhotic patients being the most promising candidate for assessing pressure-derived changes in LSECs.

### CBX7 downregulation correlates with HVPG in liver biopsies from aCLD patients

To investigate the potential role of CBX7 as a pressure-derived biomarker, the present inventors interrogated its expression in liver biopsies from two different cohorts of aCLD patients.

The discovery cohort included an array expression from healthy (n = 12) and HCV-cirrhotic patients (n=19) with similar ages, and with the majority of patients with a Child Pugh A grade (n=16). Moreover, 6 patients had an HVPG < 10 mmHg, 5 patients an HVPG between 10 and 12 mmHg, and 8 patients showed an HPVG over 12 mmHg.

Liver biopsies from the validation cohort included a higher number of patients with alcohol associated CLD (OH) (n = 25) and HCV (n = 16) aetiologies and were analyzed by qPCR.

Patients mean age was 58.0 ± 7.5 years for the aCLD group and 56.1 ± 16.4 for the healthy group, both with a similar gender distribution. Child Pugh scores were also evenly distributed, with 16, 15 and 9 patients with scores of A, B and C, respectively. Regarding HVPG, 7 patients had a measurement below 10 mmHg, 8 patients between 10 and 12 mmHg, and 21 patients above 12 mmHg.

*CBX7* gene expression was analyzed in both cohorts comparing healthy volunteers (HVPG < 5 mmHg) against patients with PH (HVPG ≥ 5 mmHg).

It was found that CBX7 was significantly downregulated in patients with PH in both cohorts, as summarized in Table 3.

Interestingly, CBX7 downregulation was aetiology independent (and significantly correlated with HVPG . AUROC curves confidence interval (CI), cut-off values, sensibility, and specificity as well as PPV and NPV for the exploratory and validation cohorts are shown in Table 4 below.

AUROC values for the discovery and validation cohorts showed excellent diagnostic ability, being both above 0.965 with a sensitivity of 100% and specificities of at least 75% and 90% respectively. Aetiology-specific analysis in the validation cohort showed a better predictive power for HCV patients (AUROC: 0.994 vs 0.945 and specificity: 93.7 vs 88) compared to those from OH aetiology. Nevertheless, in both populations, CBX7 expression was highly sensible (100%) in detecting patients with PH. *CBX7* gene expression was also assessed in a small subgroup of HCV patients after 12 months of antiviral therapy treatment and its expression was significantly higher than in non-treated HCV patients (+148% versus non-treated patients, p-value 0.0134). Thus suggesting that CBX7 reflects improvements in HVPG in response to therapy.

Collectively, these results suggest that *CBX7* is a pressure-sensitive factor in LSECs and provide the overarching rational to further dissect the mechanisms that command *CBX7* expression.

**Table 3**

| | ***Group*** | ***N*** | ***CBX7 expression*** | ***p-value*** | ***Correlation with HVPG*** | ***p-value*** |
|---|---|---|---|---|---|---|
| *Discovery cohort* | NP | 12 | 1.00 ± 0.08 | < 0.0001 | R² = 0.439 | < 0.0001 |
| | PH | 19 | 0.75 ± 0.09 | | r = -0,6629 | |
| *Validation cohort (all)* | NP | 11 | 1.00 ± 0.19 | < 0.0001 | R² =0.259 | < 0.001 |
| | PH | 42 | 0.33 ± 0.25 | | r = -0,5096 | |
| *Validation cohort (OH)* | NP | 11 | 1.00 ± 0.19 | < 0.0001 | R² =0.317 | < 0.001 |
| | PH | 26 | 0.30 ± 0.27 | | r = -0,5632 | |
| *Validation cohort (HCV)* | NP | 11 | 1.00 ± 0.19 | < 0.0001 | R² =0.393 | < 0.001 |
| | PH | 16 | 0.39 ± 0.21 | | r = -0,6269 | |

**Table 4. Performance of CBX7 liver expression to predict portal hypertension in the discovery and validation cohorts.**

| | ***n (NP*/*PH)*** | ***AUROC (95% CI)*** | ***Cut-off*** | ***Sens (%)*** | **Spec *(%)*** | ***PPV (%)*** | ***NPV (%)*** |
|---|---|---|---|---|---|---|---|
| *Discovery cohort* | 12/19 | 0.978 (0.938 to 1.000) | 0.950 | 100 | 75 | 86.36 | 100 |
| *Validation cohort (HCV + OH)* | 11/41 | 0.965 (0.920 to 1.000) | 0.677 | 100 | 90 | 97.39 | 100 |
| *Validation cohort (HCV)* | 11/16 | 0.994 (0.976 to 1.000) | 0.677 | 100 | 93.7 | 95.9 | 100 |
| *Validation cohort (OH)* | 11/25 | 0.945 (0.875 to 1.000) | 0.648 | 100 | 88 | 94.98 | 100 |

| | | | | | | | |
|---|---|---|---|---|---|---|---|
| Values of area under the ROC curve (AUROC), the 95% confidence interval (CI), the cut-off value with better sensitivity (Sens) and specificity (Spec), positive predictive value (PPV) and negative predictive value (NPV) | | | | | | | |

### CBX7 downstream targets as non-invasive biomarkers for PH

From the above findings, the inventors made a further effort in an attempt to find non-invasive biomarkers for assessing NCSPH/CSPH related to *CBX7*-regulated circulating proteins.

Thus, ECAD and SPINK1 expression was analyzed in plasma from the biomarker cohort, which included ACLD patients from three different aetiologies (HCV, OH and metabolic-associated CLD) with similar age and gender distribution. Reference values were considered from those patients with NP (HVPG < 5 mmHg) for assessing PH and from patients with an HVPG < 10 mmHg when assessing CSPH.

Plasmatic levels of ECAD and SPINK1 were significantly upregulated in patients with PH compared to patients with normal pressure (NP) (see Table 5 below).

AUROC curves to stratify patients between NP or PH were calculated for both markers alone or combined using the following formula: Y = 1 / 1 + exp(+4.503 - (0.06*ECAD(ng/ml))-(0.055*SPINK1(ng/ml)).A cut-off value of 0.579 for the combination of ECAD+SPINK1 exhibited the best AUROC (0.911) compared to those for the individual markers (0.889 and 0.747 for ECAD and SPINK1, respectively). Test performance statistics for ECAD+SPINK1 showed a 91.3% sensitivity and 83.3% specificity (Table 6 below).

When assessing CSPH, ECAD expression was significantly upregulated in patients with CSPH. AUROC curves for assessing patients below and above 10 mmHg was calculated for both markers and their combination using the formula as follows: Y = 1 / 1 + exp(+1.116 - (0.020*ECAD(ng/ml))-(0.013*SPINK1(ng/ml)).

The combination of ECAD+SPINK1 showed the best predictive power; when using a cut off of 0.647 test sensitivity was 91.4% and specificity reached 63.6%. Table 7 summarizes the results.

Based on these findings, it is proposed the determination of the plasmatic levels of ECAD and/or SPINK1, two of CBX7 targets, as non-invasive biomarkers for PH onset and CSPH monitoring in aCLD patients.

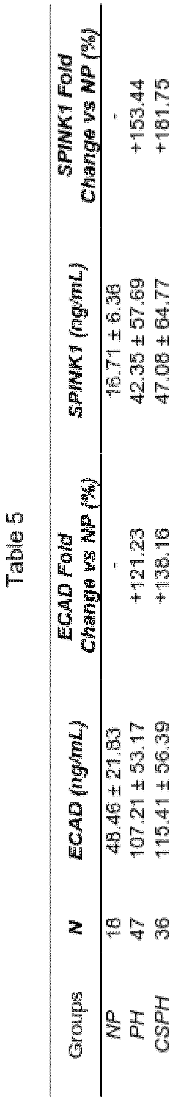

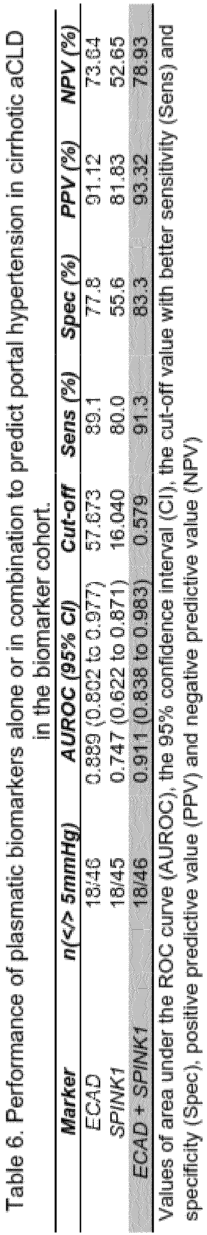

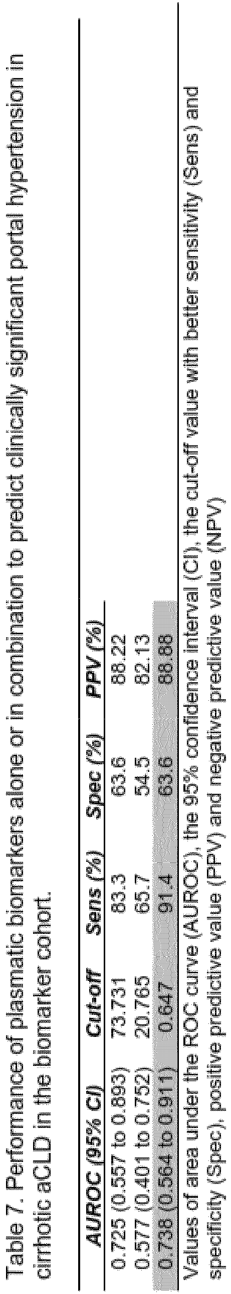

### Validation of ECAD and SPINK1 predictive capacity in another patient cohort

Plasma ECAD and SPINK1 expression were analysed as explained above in a different patient cohort in order to further validate their capacity to diagnose PH.

The cohort included 12 subjects without PH and 35 hypertensive cirrhotic patients with HP. Reference values of HVPG < 10 mmHg were considered for assessing PH. Patient details are provided in table 8 below:

**TABLE 8**

| | **NP (n=12)** | **PH (n=35)** |
|---|---|---|
| **Gender, F/M** | 7/5 | 29/6 |
| **Age, years** | | 58.6 ± 8.1 |

| **Liver disease etiology, n (%)** | | |
|---|---|---|
| OH | | 26 (74.3) |
| HCV | | 6 (17.1) |
| HBV | | 3 (8.6) |
| **HVPG, mmHg** | | |
| HVPG < 10 mmHg | | 0 |
| HVPG ≥ 10 mmHg | | 35 |
| **Child Pugh score,** | | 9 ± 1.3 |
| A/B/C | | 0/24/11 |
| **MELD score** | | 18.3 ± 3.8 |
| **AST, (IU/L)** | | 45.1 ± 20.5 |
| **ALT, (IU/L)** | | 30 ± 20.1 |
| **GGT, (IU/L)** | | 97.9 + 100.8 |

| **Alkaline phosphatase, (IU/L)** | | |
|---|---|---|
| **Bilirubin, (mg/dL)** | | 2.4 ± 1.5 |
| **Albumin, (g/L)** | | 2.9 ± 0.5 |
| **Platelet count, (103/mL)** | | 117.6 ± 65.9 |

Plasmatic levels of ECAD and SPINK1 were significantly upregulated in patients with PH compared to patients with normal pressure (NP) (Table 9).

**TABLE 9**

| **E-Cadherin** | Mean | SD | SEM | p-value (T-test) |
|---|---|---|---|---|
| NP | 65,84 | 12,53 | 3,62 | 0,0002 |
| PH | 151,34 | 72,52 | 12,26 | |

| **SPINK1** | Mean | SD | SEM | p-value (T-test) |
|---|---|---|---|---|
| NP | 25,41 | 5,21 | 1,50 | 0,0012 |
| PH | 88,95 | 63,35 | 10,71 | |

AUROC curves to stratify patients between NP or PH were calculated for both markers alone and combined using the formula above indicated.

A cut-off value of 0,50 for the combination of ECAD+SPINK1 exhibited a perfect AUROC (1.000) compared to those for the individual markers (0.962 and 0.971 for ECAD and SPINK1, respectively). Test performance statistics for ECAD+SPINK1 showed 100% sensitivity and 100% specificity (Table 10 below).

**TABLE 10**

| Marker | n (</> 5mmHg) | AUROC (95% CI) | Cut-off | Sens (%) | Spec (%) | PPV (%) | NPV (%) |
|---|---|---|---|---|---|---|---|
| ECAD | 12/35 | 0.962 (0.907 to 1.000) | 81,52 | 94,3 | 100 | 100 | 85,74 |
| SPINK1 | 12/35 | 0.971 (0.920 to 1.000) | 32,03 | 97,1 | 91,7 | 97,15 | 91,56 |
| ECAD+ SPINK1 | 12/35 | 1.000 (1.000 to 1.000) | 0,50 | 100 | 100 | 100 | 100 |

These results corroborate that ECAD or SPINK1 plasma levels are robust biomarkers for the identification of a patient suffering from PH. Furthermore, these results demonstrate that the combination of ECAD or SPINK1 is able to perfectly distinguish between NP and HP patients (100% sensitivity and 100% specificity).

### Citation List

Non Patent Literature
- Bosch, J. et al. " The management of portal hypertension: Rational basis, available treatments and future options", J. Hepatol., 2008, 48, 68-92;
- Burtis C. A. et al., 2008, Chapter 14, section "Statistical Treatment of Reference Values";
- Fernández-Iglesias, A. et al., "4 in 1: Antibody-free protocol for isolating the main hepatic cells from healthy and cirrhotic single rat livers", J. Cell. Mol. Med., 2019, 23, 877-886;
- Manicardi, N. et al., "Transcriptomic Profiling of the Liver Sinusoidal Endothelium during Cirrhosis Reveals Stage-Specific Secretory Signature", Cancers, 2021, 13(11), 2688-2704;
- Ortega-Ribera, M. et al., "Resemblance of the human liver sinusoid in a fluidic device with biomedical and pharmaceutical applications", Biotechnol. Bioeng., 2018, 115(10), 1-10; and
- Robin X. et al., 2009, "Bioinformatics for protein biomarker panel classification: What is needed to bring biomarker panels into in vitro diagnostics", Expert Review of Proteomics, 2009, 6 (6) p. 675-689.

## Claims

1. A method of diagnosis portal hypertension (PH) in a subject, the method comprising the steps of:
(a) measuring the amount of the gene expression product of the marker E-cadherin (ECAD), or of the markers ECAD and serine peptidase inhibitor Kazal type I (SPINK1), in an isolated fluid sample from the subject;
(b) comparing the amount with a reference value; and
(c) diagnosing the portal hypertension when the measured amount is higher than the reference value,
wherein the reference value is derived from subject/s wherein the condition PH is absent.

2. A method of prognosis PH, the method comprising measuring the amount of the gene expression product of the marker ECAD or of the markers ECAD and SPINK1, in an isolated fluid sample from the subject, at least at two different temporal points; wherein if there is an increase in the amount of expression product in the last temporal point with respect the previous one, this indicates that there is a bad prognosis, particularly this is indicative that the subject is in risk of progressing to severe portal hypertension or decompensation.

3. A method for stratifying a subject as normal portal pressure, as suffering from non-clinically significant PH (NCSPH) or as suffering clinically significant PH (CSPH), the method comprising the steps of:
(i) measuring the amount of gene expression product of the marker E-cadherin (ECAD) or of the markers ECAD and serine peptidase inhibitor Kazal type I (SPINK1) in an isolated fluid test sample of the subject;
(ii) obtaining reference control values V1 and V2, wherein:
V1 is calculated measuring the amount of the marker(s) in a group of samples from subjects with a portal vein pressure equal or lower than 5 mmHg; and
V2 is calculated measuring the amount of the marker(s) in a group of samples from subjects with a portal pressure equal or lower than 10 mmHg, and higher than 5 mmHg;
(iii) comparing the measured amount of step (i) with the reference control values V1 and V2, wherein:
if the measured value is equal or below V1, the subject is identified as "normal portal pressure";
if the measured value is between V1 and V2, the subject is identified as suffering NCSPH; and
if the measured value is equal or above V2, then the subject is identified as suffering CSPH.

4. A method of deciding or recommending whether to initiate a medical regimen of a subject suspicious of suffering PH, which method comprises the steps of:
Step a:
a.1) diagnosing the subject as suffering PH following the method as defined in claim 1; or, alternatively,
a.2) prognosing the subject suffering PH following the method as defined in claim 2; or, alternatively,
a.3) stratifying the subject as NCSPH or CSPH following the method as defined in claim 3, and
Step b: deciding or recommending to initiate the medical regimen if the subject is diagnosed as suffering PH, or alternatively as having a bad prognosis, or alternatively as stratified as suffering from NCSPH or CSPH.

5. A method for determining the efficacy of a medical regimen in a patient already diagnosed of PH, the method comprising the steps of:
(a) measuring the amount of the gene expression product of the marker ECAD, or of the markers ECAD and SPINK1 in an isolated fluid sample from the patient prior to the administration of the medical regimen;
(b) measuring the amount of the gene expression product of the one or both markers in an isolated fluid sample from the patient once started the administration of the medical regimen; and
(c) comparing the levels measured in steps (a) and (b), in such a way that if the amount measured in step (b) is lower than the amount measured in step (a), it is indicative that the medical regimen is effective in the treatment of the condition.

6. The method of any one of the preceding claims, wherein the amount of both, the ECAD and SPINK1 markers, is determined.

7. The method of any one of the preceding claims, wherein the fluid test sample is selected from the group consisting of whole blood, plasma, serum, urine and saliva.

8. The method of any one of the preceding claims, wherein the gene expression product is protein.

9. The method of any one of the preceding claims, wherein the measuring of the amount of the gene expression product in step (a) is performed by immunochemistry.

10. The method according to claim 9, wherein the level of expression of protein is determined using an antibody or a fragment thereof able to bind to the protein.

11. The method according to claim 10, wherein said antibody or fragment thereof forms part of a kit, particularly an ELISA kit.

12. Use of ECAD alone or in combination with SPINK1 in the diagnosis and/or prognosis of PH; in the stratification of a subject as normal portal pressure, as suffering from non-clinically significant PH (NCSPH) or as suffering clinically significant PH (CSPH); in a method of deciding or recommending whether to initiate a medical regimen of a subject suspicious of suffering PH; or for determining the efficacy of a medical regimen in a patient already diagnosed of PH.

13. Use of means for performing any of the methods as defined in claims 1 to 11.

14. The use of claim 13, wherein the means are antibodies or fragments thereof.

15. The use of any one of the claims 13-14, wherein the means form part of a kit, particularly an ELISA kit.

## Patentansprüche

1. Ein Verfahren zur Diagnose von portaler Hypertonie (PH) bei einem Patienten, wobei das Verfahren die folgenden Schritte umfasst:
(a) Messen der Menge des Genexpressionsprodukts des Markers E-Cadherin (ECAD), oder der Marker ECAD und des Serinpeptidase-Inhibitors Kazal Typ I (SPINK1) in einer isolierten flüssigen Probe des Patienten;
(b) Vergleichen dieser Menge mit einem Referenzwert; und
(c) Diagnostizieren der portalen Hypertonie, wenn die gemessene Menge höher als der Referenzwert ist, wobei der Referenzwert von dem bzw. den Patienten abgeleitet wird, bei dem bzw. denen die Erkrankung PH fehlt.

2. Ein Verfahren zur Prognose von PH, wobei das Verfahren das Messen der Menge des Genexpressionsprodukts des Markers ECAD, oder der Marker ECAD und SPINK1 in einer isolierten flüssigen Probe des Patienten an mindestens zwei verschiedenen Zeitpunkten umfasst; wobei, wenn es eine Zunahme der Menge des Expressionsprodukts im letzten Zeitpunkt in Bezug auf den vorherigen gibt, dies auf eine schlechte Prognose hindeutet, insbesondere dies darauf hindeutet, dass für den Patienten das Risiko besteht, eine schwere portale Hypertonie oder eine Dekompensation zu entwickeln.

3. Ein Verfahren zum Einstufen eines Patienten als Patient mit normalem Portaldruck, als Patient, der an nicht-klinisch signifikanter PH (NCSPH, *non-clinically significant portal hypertension*) leidet, oder als Patient, der an klinisch signifikanter PH (CSPH, *clinically significant portal hypertension*) leidet, wobei das Verfahren die folgenden Schritte umfasst:
(i) Messen der Menge des Genexpressionsprodukts des Markers E-Cadherin (ECAD) oder der Marker ECAD und des Serinpeptidase-Inhibitors Kazal Typ I (SPINK1) in einer isolierten flüssigen Testprobe des Patienten;
(ii) Erhalten von den Referenzkontrollwerten V1 und V2, wobei:
V1 berechnet wird, indem die Menge des Markers bzw. der Marker in einer Gruppe von Proben aus Patienten mit einem Pfortaderdruck von 5 mmHg oder weniger gemessen wird; und
V2 berechnet wird, indem die Menge des Markers bzw. der Marker in einer Gruppe von Proben aus Patienten mit einem Portaldruck von 10 mmHg oder weniger und von mehr als 5 mmHg gemessen wird;
(iii) Vergleichen der gemessenen Menge von Schritt (i) mit den Referenzkontrollwerten V1 und V2, wobei:
wenn der gemessene Wert gleich V1 ist oder darunter liegt, der Patient als Patient mit "normalem Portaldruck" identifiziert wird;
wenn der gemessene Wert zwischen V1 und V2 liegt, der Patient als an NCSPH leidend identifiziert wird; und
wenn der gemessene Wert gleich V2 ist oder darüber liegt, der Patient als an CSPH leidend identifiziert wird.

4. Ein Verfahren zum Entscheiden oder zum Empfehlen, ob eine medizinische Behandlung eines Patienten eingeleitet werden soll, bei dem der Verdacht besteht, an PH zu leiden, wobei das Verfahren die folgenden Schritte umfasst:
Schritt a:
a.1) Diagnostizieren, dass der Patient an PH leidet, nach dem Verfahren wie in Anspruch 1 definiert; oder alternativ,
a.2) Prognostizieren, dass der Patient an PH leidet, nach dem Verfahren wie in Anspruch 2 definiert; oder alternativ,
a.3) Einstufen des Patienten als Patient mit NCSPH oder CSPH nach dem Verfahren wie in Anspruch 3 definiert, und
Schritt b: Entscheiden oder Empfehlen, die medizinische Behandlung einzuleiten, wenn es bei dem Patienten eine PH Diagnose erfolgt, oder alternativ eine schlechte Prognose festgestellt wird oder, alternativ, wenn der Patient als an NCSPH oder CSPH leidender Patient eingestuft wird.

5. Ein Verfahren zum Bestimmen der Wirksamkeit einer medizinischen Behandlung bei einem Patienten, bei dem bereits eine PH diagnostiziert wurde, wobei das Verfahren die folgenden Schritte umfasst:
(a) Messen der Menge des Genexpressionsprodukts des Markers ECAD, oder der Marker ECAD und SPINK1 in einer isolierten flüssigen Probe des Patienten vor der Verabreichung der medizinischen Behandlung;
(b) Messen der Menge des Genexpressionsprodukts des einen oder der beiden Marker in einer isolierten flüssigen Probe des Patienten nach Beginn der Verabreichung der medizinischen Behandlung; und
(c) Vergleichen der in den Schritten (a) und (b) gemessenen Werte derart, dass, wenn die in Schritt (b) gemessene Menge geringer als die in Schritt (a) gemessene Menge ist, dies darauf hindeutet, dass die medizinische Behandlung bei der Behandlung der Erkrankung wirksam ist.

6. Das Verfahren von einem der vorhergehenden Ansprüche, wobei die Menge von beiden Markern, ECAD und SPINK1, bestimmt wird.

7. Das Verfahren nach einem der vorhergehenden Ansprüche, wobei die flüssige Testprobe ausgewählt ist aus der Gruppe bestehend aus Vollblut, Plasma, Serum, Urin und Speichel.

8. Das Verfahren nach einem der vorhergehenden Ansprüche, wobei das Genexpressionsprodukt ein Protein ist.

9. Das Verfahren von einem der vorhergehenden Ansprüche, wobei das Messen der Menge des Genexpressionsprodukts in Schritt (a) durch Immunchemie durchgeführt wird.

10. Das Verfahren nach Anspruch 9, wobei der Expressionsgrad des Proteins unter Verwendung eines Antikörpers oder eines Fragments davon bestimmt wird, der bzw. das an das Protein binden kann.

11. Das Verfahren nach Anspruch 10, wobei der Antikörper oder das Fragment davon Teil eines Kits, insbesondere eines ELISA-Kits, ist.

12. Verwendung von ECAD allein oder in Kombination mit SPINK1 bei der Diagnose und/oder Prognose von PH; bei der Einstufung eines Patienten als Patient mit normalem Portaldruck, als an nicht-klinisch signifikanter PH (NCSPH) leidend oder als an klinisch signifikanter PH (CSPH) leidend; bei einem Verfahren zur Entscheidung oder Empfehlung, ob eine medizinische Behandlung eines Patienten eingeleitet werden soll, bei dem der Verdacht auf PH besteht; oder zur Bestimmung der Wirksamkeit einer medizinischen Behandlung bei einem Patienten, bei dem bereits PH diagnostiziert wurde.

13. Verwendung von Mitteln zur Durchführung eines der in den Ansprüchen 1 bis 11 definierten Verfahren.

14. Die Verwendung von Anspruch 13, wobei die Mittel Antikörper oder Fragmente davon sind.

15. Die Verwendung nach einem der Ansprüche 13 bis 14, wobei die Mittel Teil eines Kits, insbesondere eines ELISA-Kits, sind.

## Revendications

1. Un procédé de diagnostic de l'hypertension portale (HTP) chez un sujet, le procédé comprenant les étapes consistant à :
(a) mesurer la quantité du produit d'expression génique du marqueur E-cadhérine (ECAD), ou des marqueurs ECAD et inhibiteur de la sérine peptidase Kazal de type I (SPINK1), dans un échantillon de fluide isolé du sujet ;
(b) comparer ladite quantité à une valeur de référence ; et
(c) diagnostiquer l'hypertension portale lorsque la quantité mesurée est supérieure à la valeur de référence, dans lequel la valeur de référence est dérivée de sujet(s) dans lequel/lesquels l'affection HTP est absente.

2. Un procédé de pronostic de la HTP, le procédé comprenant la mesure de la quantité du produit d'expression génique du marqueur ECAD ou des marqueurs ECAD et SPINK1, dans un échantillon de fluide isolé du sujet, à au moins deux moments différents ; dans lequel si l'on observe une augmentation de la quantité du produit d'expression au dernier moment par rapport au précédent, cela indique un mauvais pronostic, en particulier cela indique que le sujet présente un risque d'évolution vers une hypertension portale sévère ou une décompensation.

3. Un procédé permettant de classer un sujet selon qu'il présente une pression portale normale, une HTP sans signification clinique (NCSPH, *non-clinically significant portal hypertension*) ou une HTP cliniquement significative (CSPH, *clinically significant portal hypertension*), ledit procédé comprenant les étapes consistant à :
(i) mesurer la quantité de produit d'expression génique du marqueur E-cadhérine (ECAD) ou des marqueurs ECAD et inhibiteur de la sérine peptidase Kazal de type I (SPINK1) dans un échantillon de fluide à analyser isolé du sujet ;
(ii) obtenir des valeurs de contrôle de référence V1 et V2, où :
V1 est calculé en mesurant la quantité du ou des marqueurs dans un groupe d'échantillons provenant de sujets ayant une pression de la veine porte égale ou inférieure à 5 mmHg ; et
V2 est calculé en mesurant la quantité du ou des marqueurs dans un groupe d'échantillons provenant de sujets ayant une pression portale égale ou inférieure à 10 mmHg et supérieure à 5 mmHg ;
(iii) comparer la quantité mesurée de l'étape (i) avec les valeurs de contrôle de référence V1 et V2, où :
si la valeur mesurée est égale ou inférieure à V1, le sujet est identifié comme présentant une « pression portale normale » ;
si la valeur mesurée est d'entre V1 et V2, le sujet est identifié comme souffrant de NCSPH ; et
si la valeur mesurée est égale ou supérieure à V2, alors le sujet est identifié comme souffrant de CSPH.

4. Un procédé pour décider ou de recommander s'il convient d'instaurer un traitement médical chez un sujet suspecté de souffrir de HTP, lequel procédé comprend les étapes consistant à :
Étape a :
a.1) diagnostiquer que le sujet souffre de HTP selon le procédé défini dans la revendication 1 ; ou, en variante,
a.2) établir un pronostic de HTP chez le sujet selon la méthode telle que définie dans la revendication 2 ; ou, en variante,
a.3) classer le sujet en tant que sujet avec NCSPH ou CSPH selon le procédé défini dans la revendication 3, et
Étape b : décider ou recommander d'initier le traitement médical si le sujet est diagnostiqué comme souffrant de HTP, ou, en variante, comme ayant un mauvais pronostic, ou, en variante, s'il est classé comme sujet souffrant de NCSPH ou de CSPH.

5. Un procédé pour déterminer l'efficacité d'un traitement médical chez un patient déjà diagnostiqué de HTP, le procédé comprenant les étapes consistant à :
(a) mesurer la quantité du produit d'expression génique du marqueur ECAD, ou des marqueurs ECAD et SPINK1 dans un échantillon de fluide isolé provenant du patient avant l'administration du traitement médical ;
(b) mesurer la quantité du produit d'expression génique de l'un ou des deux marqueurs dans un échantillon de fluide isolé du patient une fois que l'administration du traitement médical a commencé ; et
(c) comparer les niveaux mesurés aux étapes (a) et (b), de telle sorte que si la quantité mesurée à l'étape (b) est inférieure à la quantité mesurée à l'étape (a), cela indique que le traitement médical est efficace dans le traitement de l'affection.

6. Le procédé de l'une quelconque des revendications précédentes, dans lequel la quantité des deux marqueurs, ECAD et SPINK1, est déterminée.

7. Le procédé de l'une quelconque des revendications précédentes, dans lequel l'échantillon de fluide à analyser est choisi dans le groupe constitué du sang total, du plasma, du sérum, de l'urine et de la salive.

8. Le procédé de l'une quelconque des revendications précédentes, dans lequel le produit d'expression génique est une protéine.

9. Le procédé de l'une quelconque des revendications précédentes, dans lequel la mesure de la quantité du produit d'expression génique dans l'étape (a) est effectuée par immunochimie.

10. Le procédé selon la revendication 9, dans lequel le niveau d'expression de la protéine est déterminé en utilisant un anticorps ou un fragment de celui-ci capable de se lier à la protéine.

11. Le procédé selon la revendication 10, dans lequel ledit anticorps ou fragment de celui-ci fait partie d'un kit, en particulier un kit ELISA.

12. Utilisation de l'ECAD seul ou en combinaison avec le SPINK1 dans le diagnostic et/ou le pronostic de la HTP ; dans le classement d'un sujet en tant que sujet avec pression portale normale, en tant que patient souffrant de HTP sans signification clinique (NCSPH) ou en tant que souffrant de HTP cliniquement significative (CSPH) ; dans un procédé permettant de décider ou de recommander s'il convient d'instaurer un traitement médical chez un sujet suspecté de souffrir de HTP ; ou pour déterminer l'efficacité d'un traitement médical chez un patient déjà diagnostiqué de HTP.

13. Utilisation de moyens pour mettre en oeuvre l'un quelconque des procédés définis dans les revendications 1 à 11.

14. L'utilisation de la revendication 13, dans laquelle les moyens sont des anticorps ou des fragments de ceux-ci.

15. L'utilisation de l'une quelconque des revendications 13-14, dans laquelle les moyens font partie d'un kit, en particulier un kit ELISA.
